# EUROPÄISCHE PATENTSCHRIFT

(11) **EP 4 277 527 B1**
(45) Veröffentlichungstag und Bekanntmachung des Hinweises auf die Patenterteilung: **25.03.2026**
(21) Anmeldenummer: 22701537.7
(22) Anmeldetag: 11.01.2022
(51) Int. Cl.: A61B 5/15

(54) **PROBENAUFNAHMEVORRICHTUNG**
SAMPLE-RECEIVING DEVICE
DISPOSITIF DE RÉCEPTION D'ÉCHANTILLON

(30) Priorität: 12.01.2021 DE 102021200214; 20.12.2021 DE 102021214685
(43) Veröffentlichungstag der Anmeldung: 22.11.2023
(73) Patentinhaber: Anvajo GmbH, 01069 Dresden (DE)
(72) Erfinder: EZKERRA FERNÁNDEZ, Aitor, 01069 Dresden (DE); HAMMER, Daniel, 01069 Dresden (DE)
(74) Vertreter: Pfenning, Meinig & Partner mbB
(86) Internationale Anmeldenummer: PCT/EP2022/050447
(87) Internationale Veröffentlichungsnummer: WO 2022/152699

(56) Entgegenhaltungen:
- WO-A2-00/74763
- US-A1- 2011 077 554
- US-A1- 2013 079 666
- US-A1- 2013 281 808

## Beschreibung

Die vorliegende Anmeldung betrifft eine Probenaufnahmevorrichtung nach dem Oberbegriff des Anspruchs 1 und ein Verfahren zur Probenaufnahme. Eine solche Probenaufnahmevorrichtung ist aus der US 2013/281808 A1 bekannt.

Im Bereich der Probenverarbeitung ist es insbesondere bei flüssigen Proben, die in kleinen Mengen aufgenommen und analysiert werden sollen, eine Herausforderung, eine passende Anzahl von Proben schnell aufzunehmen und weiterzuverarbeiten. Ein Effizienzgewinn ist hierbei durch die Verwendung sogenannter "Aliquots" möglich, also durch die Verwendung von Teilproben, ohne dass eine Qualität der Analyse negativ beeinträchtigt wird. Beispielsweise offenbart das Patent US 5,741,412 A eine Anordnung mit mehreren Kapillaren zum Aufnehmen und Analysieren der Probe. Nachteilig an derartigen Systemen ist die starre Anordnung, die zwar einen kontinuierlichen Fluss ermöglicht, aber die Flexibilität der Analyse beschränkt.

Der vorliegenden Erfindung liegt daher die Aufgabe zugrunde, eine Probenaufnahmevorrichtung vorzuschlagen, die die genannten Nachteile vermeidet, mit der also eine Probenaufnahme schnell erfolgen kann und zudem die weitere Analyse flexibel handhabbar ist.

Diese Aufgabe wird erfindungsgemäß gelöst durch eine Probenaufnahmevorrichtung nach dem Hauptanspruch 1 und ein Verfahren nach dem nebengeordneten Anspruch 11. Vorteilhafte Ausgestaltungen und Weiterbildungen sind in den abhängigen Ansprüchen beschrieben.

Die Probenaufnahmevorrichtung weist in einem Ausführungsbeispiel einen ersten Teil und einen zweiten Teil auf. Der erste Teil weist mindestens zwei über eine biegbare Verbindung miteinander verbundene stabförmige Probenaufnahmeeinheiten auf, deren von der biegbaren Verbindung wegweisende Enden in einem Grundzustand räumlich voneinander beabstandet sind. Der zweite Teil weist eine Arretiervorrichtung auf, die derart ausgebildet ist, dass die biegbare Verbindung beim Aufsetzen des ersten Teils mit seiner den Probenaufnahmeeinheiten abgewandten Seite auf den zweiten Teil durch die Form der Arretiervorrichtung gebogen wird und die Enden der mindestens zwei Probenaufnahmeeinheiten zueinander geführt werden können, so dass die in Richtung einer Probe weisend angeordneten Seiten der mindestens zwei Probenaufnahmeeinheiten unmittelbar nebeneinander angeordnet sind, durch die Arretiervorrichtung in dieser Position arretiert sind und eine einzige Probe in die mindestens zwei Probenaufnahmeeinheiten oder mit den mindestens zwei Probenaufnahmeeinheiten aufnehmbar ist.

Dadurch, dass die Probenaufnahmeeinheiten im Grundzustand einen definierten Abstand zueinander aufweisen und erst bei der passenden Translationsbewegung, d. h. dem Verbinden des ersten Teils und des zweiten Teils bzw. dem Aufsetzen des ersten Teils auf den zweiten Teil an ihren Spitzen bzw. Enden, d. h. an den der aufzunehmenden Probe zugewandten Seiten, zueinander geführt werden, ist eine klare räumliche Trennung gegeben, die aber dennoch eine schnelle Probenaufnahme nach dem Durchführen der Bewegung ermöglicht. Durch die biegbare bzw. gelenkige Verbindung der Probenaufnahmeeinheiten, die auch flexibel ausgeführt sein kann, also biegbar und dehnbar, ist die Bewegung der Probenaufnahmeeinheiten reproduzierbar möglich. Das Führen der Enden bzw. Spitzen der Probenaufnahmeeinheiten zueinander wird typischerweise durch ein Abwinkeln der Probenaufnahmeeinheiten erreicht.

Der erste Teil kann auch als Oberteil und der zweite Teil als Unterteil bezeichnet werden, es kann aber auch der erste Teil als Unterteil und der zweite Teil als Oberteil bezeichnet werden. Die von der biegbaren Verbindung wegweisenden Enden der Probenaufnahmeeinheiten sind in der Regel nach unten, d. h. in Richtung Erdmittelpunkt orientiert. Die einzelne Probe, die von den Probenaufnahmeeinheiten aufgenommen wird (d. h. die Probenaufnahmeeinheiten sind zum Aufnehmen einer Probe ausgebildet), kann sowohl gänzlich von den Probenaufnahmeeinheiten aufgenommen werden, es kann aber auch vorgesehen sein, lediglich Teilvolumina der Probe aufzunehmen. Der zweite Teil kann lediglich aus der Arretiervorrichtung bestehen, es kann aber auch vorgesehen sein, dass neben der Arretiervorrichtung noch weitere Elemente Bestandteil des zweiten Teils der Probenaufnahmevorrichtung sind, beispielsweise eine Halteeinheit zum Greifen und Halten des zweiten Teils.

Die Spitzen der Probenaufnahmeeinheiten bzw. die Enden der Probenaufnahmeeinheiten befinden sich nach erfolgtem Aufsetzen des ersten Teils auf den zweiten Teil und Biegen bzw. Abwinkeln der biegbaren Verbindung vorzugsweise in direktem, d. h. unmittelbar berührendem, Kontakt miteinander. Unter dem Begriff "unmittelbar berührender Kontakt" soll hierbei neben einem Abstand von 0 mm auch ein Abstand von bis zu 2 mm, typischerweise ein geringer Abstand von 0,1 mm bis 1 mm der Spitzen der Probenaufnahmeeinheiten zueinander verstanden werden.

Es kann vorgesehen sein, dass die biegbare Verbindung, die auch als gelenkige Verbindung bezeichnet werden kann, eine scharnierförmige Verbindung ist. Alternativ kann diese Verbindung auch als Gelenkverbindung, vorzugsweise als Festkörpergelenkverbindung bzw. Drehgelenkverbindung, ausgestaltet sein. Typischerweise sind die Probenaufnahmeeinheiten und die Verbindung stoffschlüssig ausgeführt, d. h. als ein einziges Bauteil bzw. einteilig. Alternativ können die Probenaufnahmeeinheiten aber auch jeweils als Einzelteile existieren und durch eine formschlüssige Verbindung miteinander in Kontakt gebracht und verbunden werden. Außerdem kann auch vorgesehen sein, dass eine Klebeverbindung zwischen den als einzelnen Einheiten ausgeführten Probenaufnahmeeinheiten vorliegt, so dass durch die Klebeverbindung miteinander verbunden sind. Hierdurch wird eine einfach herstellbare und einfach zu modifizierende Verbindung geschaffen.

Die biegbare Verbindung kann mittig zwischen den zwei Probenaufnahmeeinheiten angeordnet sein bzw., falls mehr als zwei Probenaufnahmeeinheiten vorgesehen sind, mittig zwischen zwei benachbarten Probenaufnahmeeinheiten angeordnet sein. Somit ergibt sich eine gleichförmige Verbiegung. Es kann jedoch auch vorgesehen sein, eine asymmetrische Verbiegung zu ermöglichen, indem die biegbare Verbindung beispielsweise nicht mittig zwischen zwei der Probeaufnahmeeinheiten angeordnet ist.

Die Probenaufnahmevorrichtung weist in einem Ausführungsbeispiel ein Oberteil und ein Unterteil auf, die formschlüssig und bzw. oder kraftschlüssig miteinander verbindbar sind. Das Oberteil umfasst ein Haltegehäuse, wobei an dem Haltegehäuse und mit diesem jeweils über eine biegbare Verbindung verbunden mindestens zwei stabförmige Probenaufnahmeeinheiten oder mindestens zwei biegbare Probenaufnahmeeinheiten angeordnet sind, die in einem Grundzustand räumlich voneinander beabstandet angeordnet sind. Das Unterteil weist eine Arretiervorrichtung auf, die derart ausgebildet ist, dass die mindestens zwei Probenaufnahmeeinheiten entweder durch eine externe Kraft oder durch die Arretiervorrichtung des Unterteils zueinander geführt werden können, so dass die in Richtung einer Probe weisend angeordneten Seiten der mindestens zwei Probenaufnahmeeinheiten unmittelbar nebeneinander angeordnet sind, durch die Arretiervorrichtung in dieser Position arretiert sind und eine einzige Probe in die mindestens zwei Probenaufnahmeeinheiten oder mit den mindestens zwei Probenaufnahmeeinheiten aufnehmbar ist. Der erste Teil entspricht hierbei also dem Oberteil, der zweite Teil entspricht dem Unterteil, in weiteren Ausführungsbeispielen kann aber auch der erste Teil dem Unterteil entsprechen und der zweite Teil dem Oberteil.

Dadurch, dass die Probenaufnahmeeinheiten im Grundzustand einen definierten Abstand zueinander aufweisen und erst bei der passenden Translationsbewegung des Unterteils in Richtung des Oberteils oder des Oberteils in Richtung des Unterteils an ihren Spitzen, d. h. an den dem Haltegehäuse abgewandten Seiten, zueinander geführt werden, ist eine klare räumliche Trennung gegeben, die aber dennoch eine schnelle Probenaufnahme nach dem Durchführen der Bewegung ermöglicht. Durch die biegbare Verbindung der Probenaufnahmeeinheiten mit dem Haltegehäuse, die auch flexibel ausgeführt sein kann, also biegbar und dehnbar, ist die Bewegung der Probenaufnahmeeinheiten reproduzierbar möglich. Indem das Oberteil und das Unterteil formschlüssig und bzw. oder kraftschlüssig miteinander verbunden oder verbindbar sind, ist zudem eine kompakte und mechanisch stabile Anordnung gegeben, die die beschriebene Bewegung wie gewünscht ausführen kann.

Unter dem Begriff "biegbar" soll hierbei insbesondere eine Ausgestaltung der Erfindung verstanden werden, bei dem ein Biegeradius vor dem Brechen 0,25° bis 180°, vorzugsweise 0,25° bis 90°, beträgt und eine elastische, plastische, größtenteils elastische oder größtenteils plastische Verformung der Probenaufnahmeeinheiten mit geringem irreversiblen plastischen bzw. elastischen Verformungsanteil erfolgt. Es kann auch vorgesehen sein, dass die Probenaufnahmeeinheiten von dem Haltegehäuse trennbar sind, indem eine Biegung an der Verbindungsstelle, die typischerweise stegförmig ausgeführt ist, über den jeweiligen Biegeradius hinaus erfolgt, so dass die Verbindungsstelle bricht. Typischerweise ist die Verbindungstelle derart ausgestaltet, dass bei einem Abziehen des Unterteils vom Oberteil die Probenaufnahmeeinheiten wieder in ihre ursprüngliche Position, d. h. die Position vor dem Aufsetzen des Unterteils, oder eine Position, bei der die Probenaufnahmeeinheiten weiter voneinander beabstandet sind als in der Ursprungsposition oder der Position, in der die Probenaufnahmeeinheiten zusammengeführt waren, zurückbewegt werden, um sie leichter abtrennen zu können.

Unter dem Begriff "stabförmig" soll im Rahmen dieser Schrift insbesondere verstanden werden, dass eine Länge des damit bezeichneten Elements um mindestens 10 Prozent größer ist als dessen Breite und bzw. oder Tiefe. Speziell zylinderförmige Elemente oder hohlzylinderförmige Elemente sollen durch den Begriff "stabförmig" beschrieben sein.

Es kann vorgesehen sein, dass das Unterteil ein mit einer als Arretiervorrichtung dienenden Öffnung versehenes Gehäuse aufweist. Durch die Öffnung können die Probenaufnahmeeinheiten somit definiert räumlich fixiert werden. Das Unterteil ist hierbei typischerweise scheibenförmig oder ringförmig ausgestaltet.

Die Probenaufnahmeeinheiten können in die Öffnung des Gehäuse einführbar und derart durch die externe Kraft zueinander bewegbar sein, dass die Probenaufnahmeeinheiten in der als Arretiervorrichtung dienenden Öffnung nach dem Einführen arretiert sind, um ein sicheres Halten der Probenaufnahmevorrichtungen in einer definierten Position zu ermöglichen.

Das Unterteil kann ein mit einer als Arretiervorrichtung dienenden durchgehenden Öffnung versehenes Gehäuse aufweisen, das zumindest einen sich verjüngend ausgestalteten Teil aufweist, wobei das Unterteil in einer Translationsbewegung derart auf die mindestens zwei Probenaufnahmeeinheiten aufsetzbar ist, dass die mindestens zwei Probenaufnahmeeinheiten bei Einführen in den sich verjüngend ausgestalteten Teil des Unterteils durch die sich verjüngende Form an ihren in Richtung einer Probe weisend angeordneten Seiten zueinander geführt werden. Es kann auch vorgesehen sein, dass das Unterteil einen geradlinig verlaufenden Teil aufweist. Durch den sich verjüngend ausgebildeten Teil des Unterteils, der, falls vorgesehen und das Unterteil nicht nur aus einem sich in Richtung der Öffnung verjüngenden Teil gebildet ist, von dem Haltegehäuse einen größeren räumlichen Abstand aufweist als der geradlinig verlaufende Teil des Unterteils, wird außerdem eine Führung der Probenaufnahmeeinheiten gewährleistet. Dadurch, dass die Spitzen der Probenaufnahmeeinheiten sich nach dem Durchführen der Translationsbewegung in der Regel im direkten Kontakt, d. h. unmittelbar berührenden Kontakt, miteinander befinden, typischerweise zumindest im Bereich der Spitzen, können diese durch die Öffnung des Unterteils geführt werden und auch vergleichsweise kleine Probenvolumen aufnehmen. Unter dem Begriff "unmittelbar berührender Kontakt" soll hierbei neben einem Abstand von 0 mm auch ein Abstand von bis zu 2 mm, typischerweise ein geringer Abstand von 0,1 mm bis 1 mm der Spitzen der Probenaufnahmeeinheiten zueinander verstanden werden.

Das Unterteil kann einen sich konisch verjüngenden Teil, typischerweise einen sich konisch verjüngenden unteren Teil, aufweisen, um eine gleichmäßige Führung der Spitzen der Probenaufnahmeeinheiten zu gewährleisten.

Es kann vorgesehen sein, dass der geradlinig verlaufende Teil des Unterteils zum Herstellen einer Betriebsbereitschaft der mindestens zwei Probenaufnahmeeinheiten bei der Translationsbewegung in eine entsprechende Ausnehmung des Oberteils einführbar ist. Hierdurch wird eine kompakte Ausführung der Probenaufnahmevorrichtung realisiert, bei der auch die Translationsbewegung gezielt geführt werden kann durch die Aufnahme des unteren Teils. Der geradlinig verlaufende Teil des Unterteils kann auch derart ausgestaltet sein, dass er sich ebenfalls in Richtung des sich verjüngend ausgestalteten Teils verjüngt, wobei allerdings die Verjüngung geringer ist als bei dem sich verjüngend ausgebildeten Teils des Unterteils. Als "geradlinig" soll dabei eine Verjüngung um bis zu 5° gelten.

Eine Länge des geradlinig verlaufenden Teils des Unterteils kann einer Länge der Probenaufnahmeeinheiten entsprechen, so dass im Grundzustand die Probenaufnahmeeinheiten im unteren Teil bzw. Unterteil beabstandet zueinander angeordnet sind und bei Beginn der Translationsbewegung durch den Kontakt mit dem sich verjüngend ausgestalteten Teil sogleich mit ihren Spitzen zusammengeführt werden. Alternativ oder zusätzlich kann die Länge der Probenaufnahmeeinheiten so gewählt sein, dass die in Richtung der Probe weisende Seite der Probenaufnahmeeinheiten im Bereich der Öffnung, die an dem Unterteil vorhanden ist, angeordnet ist. Unter dem Bereich der Öffnung soll hierbei insbesondere der Bereich verstanden werden, der von der Öffnung einen Abstand aufweist, der geringer als die Länge der Öffnung ist.

Das Unterteil ist typischerweise in einer im Haltegehäuse des Oberteils angeordneten Führung bewegbar geführt ist und vorzugsweise durch mindestens einen Anschlag in seiner Bewegungsfähigkeit begrenzt ist. Somit ergibt sich eine klar definierte Bewegung um die Probenaufnahmevorrichtung vom Grundzustand, in dem Oberteil und Unterteil nicht zusammengeschoben sind, in den zusammengeschobenen Zustand zu überführen. Es kann auch vorgesehen sein, dass das Unterteil und das Oberteil durch eine Steckverbindung miteinander verbunden sind. Typischerweise sind das Unterteil und das Oberteil aus einem identischen Werkstoff ausgebildet, es kann aber auch vorgesehen sein, dass verschiedene Werkstoffe für das Unterteil und das Oberteil verwendet werden. Zudem können der erste Teil und der zweite Teil des Unterteils gleich lang sein.

Es kann auch vorgesehen sein, eine, typischerweise zweiteilig ausgestaltete, Haltevorrichtung als Arretiervorrichtung zu verwenden, durch die die durch die externe Kraft bewegten mindestens zwei Probenaufnahmeeinheiten in ihrer Position eingeklemmt sind. Die Haltevorrichtung wird hierzu vorzugsweise an die oder in die Verbindungen angebracht.

Es kann vorgesehen sein, dass zumindest eine der Probenaufnahmeeinheiten als Kapillare, Röhre, Faser, vorzugsweise Hohlfaser, Tupfer, Bürste, Spatel, Spachtelmesser, Nadel, Membran, Schaufel, Löffel, Schwamm, vorzugsweise fester Schwamm, oder Stab, vorzugsweise schraubenförmiger Stab, oder mit einer Kapillare, einer Röhre, einer Faser, vorzugsweise einer Hohlfaser, einem Tupfer, einer Bürste, einem Spatel, einem Spachtelmesser, einer Nadel, einer Membran, einer Schaufel, einem Löffel, einem Schwamm, vorzugsweise einem festen Schwamm, oder einem Stab, vorzugsweise einem schraubenförmigen Stab, ausgebildet ist. Somit sind unterschiedliche Arten der Probenaufnahme möglich, die an den jeweiligen Verwendungszweck angepasst werden können. Die Probe selbst kann in flüssiger Form, als Festkörper, aber auch als Gel vorliegen. Durch die stabförmig bzw. länglich ausgebildeten Probenaufnahmeeinheiten kann dabei die Bewegung in Richtung der Öffnung vollzogen werden und gleichzeitig verbleibt ausreichend Volumen, um die Probe aufzunehmen.

Das Oberteil oder der erste Teil kann genau drei Probenaufnahmeeinheiten aufweisen, die in einer Reihenanordnung angeordnet sind und von denen die dem Haltegehäuse abgewandten Seiten der beiden randseitig angeordneten Probenaufnahmeeinheiten im Grundzustand in einer ersten Ebene angeordnet sind und die dem Haltegehäuse abgewandte Seite der mittig angeordneten Probenaufnahmeeinheit im Grundzustand gegenüber den beiden seitlich angeordneten Probenaufnahmeeinheiten in einer von der ersten Ebene verschiedenen, näher an dem Haltegehäuse befindlichen zweiten Ebene angeordnet ist. Insbesondere bei einer entsprechenden Längenanpassung der Probenaufnahmeeinheiten kann somit eine kompakte, da platzsparende Anordnung erreicht werden, bei der nach Durchführen der Translationsbewegung, also im zusammengeschobenen Zustand, alle Probenaufnahmeeinheiten in einer Ebene enden, also auf gleicher Höhe enden. Als Reihenanordnung soll hierbei insbesondere jede Anordnung angesehen werden, bei der in Draufsicht auf die Spitzen, d. h. auf die der Probe zugewandten bzw. dem Haltegehäuse abgewandten Seiten ein Dreieck gebildet wird, bei dem der größte Innenwinkel mindestens 120° beträgt. Es kann also sowohl vorgesehen sein, dass alle drei Probenaufnahmeeinheiten in seitlicher Ansicht in einer Ebene angeordnet sind als auch, dass zumindest eine der Probenaufnahmeeinheit in einer zu der Ebene der beiden anderen Probenaufnahmeeinheiten versetzt angeordneten Ebene positioniert ist.

Das Unterteil kann ein die Öffnung überdeckendes volumenveränderliches Säuberungselement aufweisen, vorzugsweise einen Schwamm, in das die mindestens zwei Probenaufnahmeeinheiten einbringbar sind. Alternativ kann auch ein die Öffnung überdeckendes volumenveränderliches Säuberungselement auf die mindestens zwei Probenaufnahmeeinheiten aufsetzbar sein. Somit kann nach jeder Probenaufnahme eine Säuberung zumindest der Außenseiten der Probenaufnahmeeinheiten durchgeführt werden. Das Säuberungselement wird dabei typischerweise mit dem Unterteil verschoben und gleitet somit an den Außenseiten der Probenaufnahmeeinheiten entlang. Im Idealfall werden diese Außenseiten einmal der Länge nach abgefahren. Das Säuberungselement kann aus Polyvinylchlorid (PVC), typischerweise PVC-Schaum, Polyurethan (PU), typischerweise PU-Schaum, Acrylnitril-Butadien-Kautschuk-Schaum, Ethylen-vinyl-Azetat-Schaum (EVA), Weichpolyethylen (low-density polyethylene, LDPE), typischerweise LDPE-Schaum, Neopren, expandiertem Polystyren, Silikonkautschukschaum, Polypropylenschaum,Polyterephthalatschaum, Zellulose, Nitrozellulose oder Baumwolle ausgebildet sein oder die genannten Werkstoffe zumindest umfassen.

Die Öffnung selbst ist vorzugsweise länglich ausgestaltet, d. h. ihre Länge ist größer als ihre Breite. Hierdurch können die Probenaufnahmeeinheiten einfacher in die Öffnung eingeführt werden. Alternativ kann die Öffnung auch rund sein bzw. abgerundete Ecken aufweisen.

Das Haltegehäuse oder der erste Teil kann an einer der in Richtung der Probe weisend angeordneten Seiten der mindestens zwei Probenaufnahmeeinheiten abgewandten Seite geschlossen ausgebildet ist, um die mechanische Stabilität zu erhöhen und eine ungewollte Beeinflussung der in den Probenaufnahmeeinheiten enthaltenen Probe zu verhindern oder eine Barriere für die aufgenommene Probe zu bilden.

Die Arretiervorrichtung des zweiten Teils kann eine trichterförmige Form zum Aufnehmen des ersten Teils aufweisen. Es kann alternativ oder zusätzlich vorgesehen sein, dass der erste Teil und der zweite Teil formschlüssig und bzw. oder kraftschlüssig miteinander verbindbar bzw. verbunden sind. Indem die beiden Teile formschlüssig und bzw. oder kraftschlüssig miteinander verbunden oder verbindbar sind, ist zudem eine kompakte und mechanisch stabile Anordnung gegeben, die die beschriebene Bewegung wie gewünscht ausführen kann.

Zumindest der erste Teil samt den mindestens zwei Probenaufnahmeeinheiten kann aus einem für elektromagnetische Strahlung im optisch sichtbaren Wellenlängenbereich, d. h. im Wellenlängenbereich zwischen 400 nm und 700 nm, durchlässigen Kunststoff ausgebildet sein bzw. aus diesem bestehen. Unter "durchlässig" soll in diesem Fall verstanden werden, dass mindestens 90 Prozent der auftreffenden elektromagnetischen Strahlung durch das Bauteil transmittiert werden, so dass ein Benutzer einen Füllstand der Probenaufnahmeeinheiten optisch kontrollieren kann. Dies ist besonders vorteilhaft in Situationen, in denen einen korrekte Probenaufnahme erschwert sein kann (beispielsweise bei der Blutabnahme an der Fingerkuppe). Typischerweise sind der erste Teil und der zweite Teil aus einem identischen Werkstoff ausgebildet, es kann aber auch vorgesehen sein, dass verschiedene Werkstoffe für den ersten Teil und den zweiten Teil verwendet werden.

Die Probenaufnahmeeinheit kann bzw. das Oberteil oder der erste Teil und das Unterteil oder der zweite Teil können aus einem Polymer, einem Metall, Gummi, Glas oder einem Elastomer ausgebildet sein oder diesen Werkstoff zumindest umfassen. Das Oberteil und das Unterteil sind dabei typischerweise aus dem gleichen Werkstoff ausgebildet, es können aber auch verschiedene Werkstoffe für verschiedene Teile oder Einheiten verwendet werden. Vorzugsweise ist die Probenaufnahmeeinheit, d. h. das Oberteil und das Unterteil, aus Polypropylen (PP), Acrylnitril-Butadien-Styrol-Copolymer (ABS), Polystyrol (PS), Cycloolefin-Copolymer (COC), Cycloolefin-Polymer (COP), Polyethylenterephthalat (PET), Polyvinylchlorid (PVC), Hartpolyethylen (high-density polyethylene, HDPE) oder Weichpolyethylen (low-density polyethylene, LDPE), Polyvinylchlorid (PVC), typischerweise PVC-Schaum, Polyurethan (PU), typischerweise PU-Schaum, Acrylnitril-Butadien-Kautschuk-Schaum, Ethylen-vinyl-Azetat-Schaum (EVA), Weichpolyethylen (low-density polyethylene, LDPE), typischerweise LDPE-Schaum, Neopren, expandierdem Polystyren, Silikonkautschukschaum, Polypropylenschaum oder Polyterephthalatschaum ausgebildet sein oder einen der genannten Werkstoffe wenigstens umfassen. Typischerweise ist jedoch nur das Unterteil aus einem Schaum, in der Regel einem der zuvor genannten Schäume, oder einem Schaum und einem der genannten Polymere ausgebildet oder besteht aus Schaum oder Schaum und einem der genannten Polymere, d. h. das Oberteil ist vorzugsweise aus den genannten Polymerwerkstoffen mit Ausnahme der Schäume herstellbar bzw. hergestellt.

Die Erfindung betrifft außerdem ein Ausführungsbeispiel eines Verfahren zur Probenaufnahme mittels der beschriebenen Probenaufnahmevorrichtung, bei dem die mindestens zwei Probenaufnahmeeinheiten entweder durch eine externe Kraft oder durch die Arretiervorrichtung des Unterteils zueinander geführt werden, so dass die in Richtung einer Probe weisend angeordneten Seiten der mindestens zwei Probenaufnahmeeinheiten unmittelbar nebeneinander angeordnet sind, durch die Arretiervorrichtung in dieser Position arretiert sind und eine einzige Probe in die mindestens zwei Probenaufnahmeeinheiten oder mit den mindestens zwei Probenaufnahmeeinheiten aufgenommen wird.

Bei einem Verfahren zur Probenaufnahme kann insbesondere das Unterteil der beschriebenen Probenaufnahmevorrichtung in Bezug auf das Oberteil derart bewegt werden, dass die mindestens zwei Probenaufnahmeeinheiten in den sich verjüngend ausgestalteten Teil des Unterteils eingeführt werden und in Richtung einer Probe weisend angeordneten Seiten der mindestens zwei Probenaufnahmeeinheiten zueinander geführt werden, so dass die in Richtung einer Probe weisend angeordneten Seiten der mindestens zwei Probenaufnahmeeinheiten unmittelbar nebeneinander angeordnet sind und eine einzige Probe in die mindestens zwei Probenaufnahmeeinheiten aufgenommen wird.

Die Erfindung betrifft außerdem ein Ausführungsbeispiel eines Verfahrens zur Probenaufnahme mittels der beschriebenen Probenaufnahmevorrichtung, bei dem die biegbare Verbindung beim Aufsetzen des ersten Teils mit seiner den Enden der Probenaufnahmeeinheiten abgewandten Seite auf den zweiten Teil durch die Form der Arretiervorrichtung gebogen werden und die Enden der mindestens zwei Probenaufnahmevorrichtungen zueinander geführt werden, so dass die in Richtung einer Probe weisend angeordneten Enden der mindestens zwei Probenaufnahmeeinheiten unmittelbar nebeneinander angeordnet sind, durch die Arretiervorrichtung in dieser Position arretiert sind und eine einzige Probe in die mindestens zwei Probenaufnahmeeinheiten oder mit den mindestens zwei Probenaufnahmeeinheiten aufnehmbar ist.

Ausführungsbeispiele der Erfindung sind in den Zeichnungen dargestellt und werden nachfolgend anhand der Figuren 1 bis 23 beschrieben.

Es zeigen:
- Fig. 1: eine Schnittdarstellung eines Oberteils einer Probenaufnahmevorrichtung;
- Fig. 2: eine Draufsicht auf das in Figur 1 gezeigte Oberteil;
- Fig. 3: eine Figur 1 entsprechende Darstellung des Oberteils mit zusammengeführten Probenaufnahmeeinheiten;
- Fig. 4: eine Figur 2 entsprechende Ansicht des in Figur 3 dargestellten Zustands;
- Fig. 5: eine perspektivische Ansicht eines Unterteils;
- Fig. 6: eine Schnittdarstellung des Unterteils;
- Fig. 7: eine Schnittansicht des mit dem Unterteil kombinierten Oberteils;
- Fig. 8: eine Figur 7 entsprechende Ansicht, bei der das Oberteil und das Unterteil ineinander geschoben sind;
- Fig. 9: eine Figur 5 entsprechende Ansicht mit einen Säuberungselement;
- Fig. 10: eine Figur 8 entsprechende Ansicht mit dem Säuberungselement
- Fig. 11: eine Figur 7 entsprechende Ansicht mit einem scheibenförmigen oder ringförmigen Unterteil;
- Fig. 12: eine Figur 12 entsprechende Ansicht mit aufgesetztem Unterteil;
- Fig. 13: eine Figur 7 entsprechende Ansicht mit zweiteiligem Klemmsystem;
- Fig. 14: eine Figur 13 entsprechende Ansicht mit aufgesetztem zweiteiligen Klemmsystem;
- Fig. 15: eine Figur 12 entsprechende Ansicht mit aufgesetztem scheibenförmigem Unterteil und Säuberungselement;
- Fig. 16: eine Figur 14 entsprechende Ansicht mit aufgesetztem zweiteiligen Klemmsystem und Säuberungselement;
- Fig. 17: eine schematische Schnittdarstellung des ersten Teils und des zweiten Teils der Probenaufnahmevorrichtung im getrennten Zustand;
- Fig. 18: eine Figur 17 entsprechende Ansicht im zusammengeführten Zustand
- Fig. 19: eine Figur 17 entsprechende Darstellung eines weiteren Ausführungsbeispiels der Probenaufnahmevorrichtung;
- Fig. 20: eine Figur 18 entsprechende Darstellung des in Figur 3 gezeigten Ausführungsbeispiels;
- Fig. 21: eine den Figuren 17 und 19 entsprechende Darstellung eines weiteren Ausführungsbeispiels der Probenaufnahmevorrichtung;
- Fig. 22: eine Figur 18 entsprechende Darstellung des in Figur 19 gezeigten Ausführungsbeispiels und
- Fig. 23: ein Ausführungsbeispiel mit einem Verbindungsmechanismus.

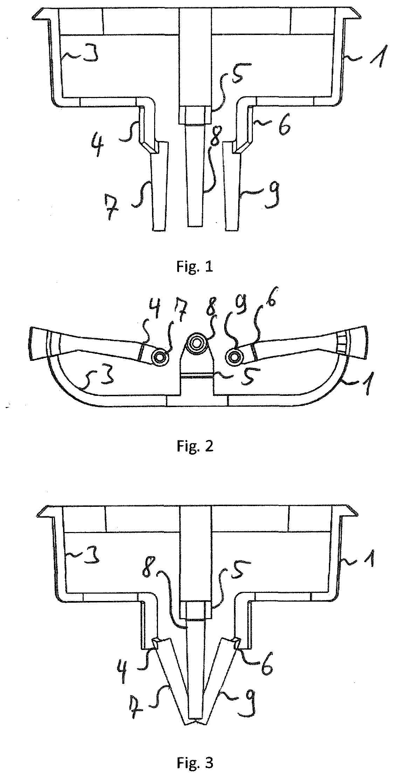

Figur 1 zeigt in einer Schnittansicht ein Oberteil 1 einer Probenaufnahmevorrichtung. Das einteilige Oberteil 1 besteht in dem dargestellten Ausführungsbeispiel aus einem Haltegehäuse 3 aus einem für elektromagnetische Strahlung im optisch sichtbaren Wellenlängenbereich zwischen 400 nm und 700 nm durchlässigen Kunststoff, d. h. mindestens 90 Prozent der auftreffenden elektromagnetischen Strahlung werden durch das Oberteil 1 transmittiert. Alternativ kann das Oberteil 1 aber auch aus einem opaken Werkstoff ausgebildet sein, der ein Transmissionsvermögen von weniger als 30°Prozent der auftreffenden elektromagnetischen Strahlung im optisch sichtbaren Wellenlängenbereich aufweist. An dem Haltegehäuse 3 sind von diesem wegweisend drei Probenaufnahmeeinheiten 7, 8, 9 angeordnet, die mit dem Haltegehäuse 3 jeweils über eine flexible Verbindung 4, 5, 6, d. h. jeweils über eine Verbindungsstelle verbunden sind.

In dem dargestellten Ausführungsbeispiel sind die Probenaufnahmeeinheiten 7, 8, 9 und das Haltegehäuse 3 aus dem gleichen Werkstoff ausgebildet, es kann jedoch auch vorgesehen sein, dass verschiedene Werkstoffe verwendet werden oder sich der verwendete Werkstoff hinsichtlich einer Eigenschaft, beispielsweise seiner Transparenz, in den verschiedenen Elementen voneinander unterscheidet.

Die Probenaufnahmeeinheiten 7, 8, 9 sind länglich bzw. stabförmig ausgebildet und in ihrem Inneren hohl, d. h. Hohlzylinder bzw. Röhren. In weiteren Ausführungsbeispielen können die Probenaufnahmeeinheiten 7, 8, 9 oder zumindest eine der Probenaufnahmeeinheiten 7, 8, 9 aber auch als Tupfer, Bürste, Spatel, Spachtelmesser, Nadel, Membran, Stab, schraubenförmiger Stab, Faser, Hohlfaser, Schaufel, Spatel, Löffel, Schwamm oder Schaum ausgebildet sein. Zudem müssen nicht alle der Probenaufnahmeeinheiten 7, 8, 9 vom gleichen Typ, es kann auch vorgesehen sein, dass wenigstens eine der Probenaufnahmeeinheiten 7, 8, 9 einen von den anderen Probenaufnahmeeinheiten 7, 8, 9 abweichenden Typ aufweist. An ihrer dem Haltegehäuse 3 abgewandten und einer aufzunehmenden und nachfolgend zu analysierenden Seite zugewandten Seite weisen die Probenaufnahmeeinheiten in dem dargestellten Ausführungsbeispiel jeweils einen geringeren Durchmesser als an ihrer dem Haltegehäuse 3 zugewandten Seite auf. Das Haltegehäuse 3 selbst ist an seiner den Probeaufnahmeeinheiten 4, 5,6 abgewandten Seite offen, kann in weiteren Ausführungsbeispielen aber auch geschlossen sein.

Über die flexiblen, d. h. biegbaren und dehnbaren Verbindungen 4, 5, 6 können die die Probeaufnahmeeinheiten 7, 8, 9 in geringem Maße geknickt bzw. rotiert werden, die Verbindungen 4, 5, 6 sind also scharnierförmig ausgestaltet. Zudem ist es auch möglich, durch ein Biegen über den jeweiligen Biegeradius hinaus die Probenaufnahmeeinheiten 7, 8, 9 von dem Haltegehäuse 3 zu trennen, d. h. die Verbindungen 4, 5, 6 aufzutrennen. Es kann auch vorgesehen sein, dass die Verbindungen 4, 5, 6 oder zumindest eine oder zwei der Verbindungen 4, 5, 6 derart ausgestaltet sind, dass sie bei einem Entfernen des Unterteils 2 wieder in die ursprüngliche Position, d. h. die in Figur 1 wiedergegebene Stellung zurückbewegt werden.

In dem in Figur 1 dargestellten Ausführungsbeispiel sind die beiden seitlich angeordneten Probenaufnahmeeinheiten 7 und 9 kürzer als die mittig angeordnete Probenaufnahmeeinheit 8 ausgestaltet, d. h. ihre Länge ist geringer als die Länge der mittigen Probenaufnahmeeinheit 8. Zudem sind die dem Haltegehäuse 3 abgewandten Enden der beiden seitlich angeordneten Probenaufnahmeeinheiten 7 und 9 in einer gemeinsamen Ebene angeordnet, wobei das Ende der mittigen Probenaufnahmeeinheit 8 nicht in dieser Ebene liegt, sondern in einer näher an dem Haltegehäuse 3 befindlichen Ebene. Die Probenaufnahmeeinheiten 7, 8, 9 können jeweils identisch ausgestaltet sein, es ist jedoch auch möglich, jeweils paarweise verschiedene Probenaufnahmeeinheiten 7, 8, 9 zu verwenden. Die Anzahl ist zudem variabel und kann neben den dargestellten drei Probenaufnahmeeinheiten 7, 8, 9 auch nur zwei Probenaufnahmeeinheiten oder mehr als zwei Probenaufnahmeeinheiten, beispielsweise bis zu zehn Probenaufnahmeeinheiten, umfassen. Die Länge der Probenaufnahmeeinheiten liegt in der Regel zwischen 1 mm und 20 mm, vorzugsweise im Bereich von 8 mm und 14 mm.

Die dargestellten Probenaufnahmeeinheiten 7, 8, 9 sind jeweils als Kapillaren für die Aufnahme von flüssigen Proben, beispielsweise Tropfen einer bestimmten zu analysierenden Flüssigkeit wie Blutstropfen von einer Fingerspitze, im Bereich von Millilitern ausgebildet und können vorzugsweise Probenvolumen von 0,25 µl bis 25 µl, besonders vorzugsweise 1 µl bis 20 µl aufnehmen. Die Probenaufnahmeeinheiten 7, 8, 9 weisen, unabhängig von der genauen Anzahl der Probenaufnahmeeinheiten 7, 8, 9, in der Regel ein jeweils identisches Innenvolumen bzw. jeweils einen innenliegenden Hohlraum mit jeweils identischem Volumen auf, d. h. es kann in jeder der Probenaufnahmeeinheiten 7, 8, 9 ein gleich großes Probenvolumen aufgenommen werden. In weiteren Ausführungsbeispielen kann aber auch das Probenvolumen wenigstens einer der Probenaufnahmeeinheiten 7, 8, 9 sich von dem Volumen der anderen Probenaufnahmeeinheiten 7, 8, 9 unterscheiden. Es können aber auch als Festkörper, beispielsweise als Granulat, Pulver oder sonstiges Schüttgut, vorliegenden Proben oder gelförmige Proben aufgenommen werden. Die Probeaufnahmeeinheiten 7, 8, 9 sind in dem gezeigten Ausführungsbeispiel zwar starr ausgeführt, können in weiteren Ausführungsbeispielen aber auch selbst biegbar sein.

In Figur 2 ist in einer Draufsicht das beschriebene Oberteil 1 dargestellt, wobei das Oberteil 1 nun von unten zu sehen ist, also quasi aus der Perspektive der aufzunehmenden Probe. Wiederkehrende Merkmale sind in dieser Figur wie auch in den folgenden Figuren mit identischen Bezugszeichen versehen. Die Probenaufnahmeeinheiten 7, 8, 9 sind in einer Reihenanordnung angeordnet, d. h. ihre unteren Spitzen bilden ein gleichschenkliges Dreieck mit einem Winkel an der Probenaufnahmeeinheit 8 größer 120°.

Figur 3 zeigt in einer Figur 1 entsprechenden Ansicht das Oberteil 1 mit zusammengeführten Probenaufnahmeeinheiten 7, 8, 9, d. h. diese sind jeweils an ihren stegförmigen Verbindungen 4, 5, 6 geknickt und mit ihren Spitzen derart aneinander geführt, dass diese sich unmittelbar berühren, also in direktem Kontakt miteinander sind. In dieser Stellung, in der auch alle Spitzen sich auf gleicher Höhe befinden, also alle Spitzen in einer einzigen Ebene enden, kann eine Probe aufgenommen werden und in jeder der Probenaufnahmeeinheiten 7, 8, 9 eine Teilprobe bzw. ein Aliquot enthalten sein.

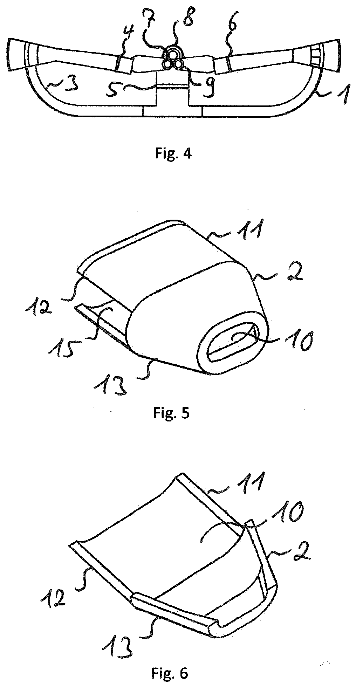

Figur 4 zeigt in einer Figur 2 entsprechenden Ansicht den in Figur 3 dargestellten zusammengeführten Zustand, bei dem praktisch eine gemeinsame Saugspitze durch die drei Spitzen der Probenaufnahmeeinheiten 7, 8, 9 gebildet wird.

Ein zu dem in den Figuren 1 bis 4 gezeigten Oberteil 1 passendes einteiliges bzw. monolithisch ausgebildetes Unterteil 2 ist in Figur 5 in einer perspektivischen Ansicht wiedergegeben. Das Gehäuse 11 des Unterteils 2 ist ebenfalls aus dem Werkstoff des Oberteils 1 und weist einen geradlinig verlaufenden ersten Teil 12 und einen mit dem ersten Teil 12 formschlüssig verbundenen, sich verjüngend ausgestalteten zweiten Teil 13 auf. Der erste Teil 12 ist in seinem Inneren hohl und weist eine Länge auf, die in dem gezeigten Ausführungsbeispiel der Länge der Probenaufnahmeeinheiten 7, 8, 9 entspricht, so dass diese in dem Innenraum des ersten Teil 12 aufgenommen werden können, ohne ihre Position zueinander zu verändern. Der erste Teil 12 kann dabei als sich in Richtung des zweiten Teils 13 verjüngend ausgebildet sein, allerdings ist dabei ein Steigungswinkel der Verjüngung geringer als ein entsprechender Steigungswinkel im zweiten Teil 13. In dem in Figur 4 dargestellten Ausführungsbeispiel beträgt der Steigungswinkel des ersten Teils 12 aufgrund seiner geradlinigen Ausgestaltung 0° (könnte aber auch bis zu 5° betragen), während der Steigungswinkel des zweiten Teils 13 45° beträgt.

Der zweite Teil 13 weist ebenfalls einen Hohlraum im Inneren und eine abschließende, längliche Öffnung 10 auf, so dass sich eine durchgehende Öffnung des Gehäuses 11, also ein zweiseitig offenes Unterteil 2 ergibt. Der zweite Teil 13 ist jedoch sich konisch in Richtung der Öffnung 10 verjüngend ausgestaltet. In dem Gehäuse 11 ist seitlich im ersten Teil 12 eine Aussparung 15 vorgesehen, die auf ein dazu korrespondierendes Führungsteil des Oberteils 1 aufsteckbar ist, so dass das Unterteil 2 entlang dieser Führung verschoben werden kann und im Oberteil 1 versenkbar ist. Beim Durchführen einer derartigen Translationsbewegung zwischen dem Oberteil 1 und dem Unterteil 2 werde die Spitzen der Probenaufnahmeeinheiten 7, 8, 9 durch den zweiten Teil 13 des Unterteils 2 zueinander bewegt und treten schließlich an der Öffnung 10 wieder aus, so dass sich eine definierte Position der Probenaufnahmeeinheiten 7, 8, 9 zur Probenaufnahme ergibt.

In Figur 6 ist zur Verdeutlichung in einer Figur 5 entsprechenden Ansicht das Unterteil 2 in Schnittansicht dargestellt. Hier ist nun der sich in Richtung der Öffnung 10 verkleinernde Durchmesser klarer zu erkennen, durch den die Spitzen der Probenaufnahmeeinheiten 7, 8, 9 zum Gleiten entlang der Innenoberfläche zueinander geführt werden. Die Innenoberfläche des zweiten Teils 13 ist hierfür glatt, d. h. stufenlos, ausgeführt, um ein widerstandsfreies Gleiten der Spitzen zu ermöglichen.

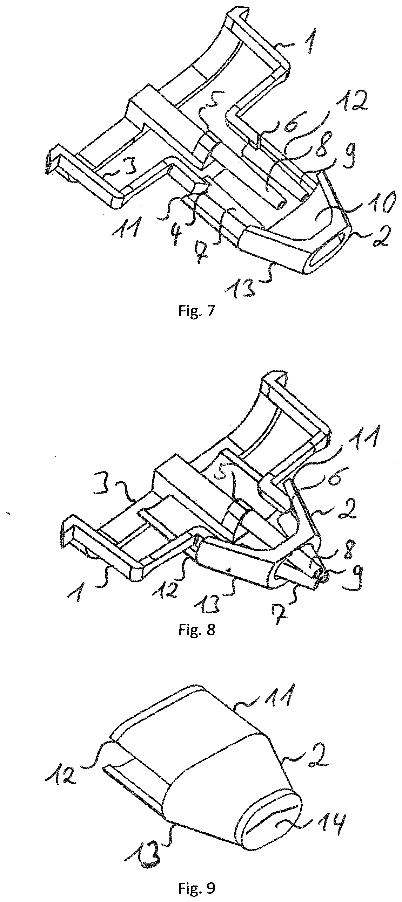

Figur 7 zeigt einen zusammengesetzten Zustand des Unterteils 2 und des Oberteils 1 in einer perspektivischen Schnittansicht. Das Unterteil 2 ist derart aufgesetzt auf das Oberteil 1, dass der erste Teil 12 die Probenaufnahmeeinheiten 7, 8, 9 gerade umschließt, allerdings nicht aus ihrer initialen Position, bei dem die Probenaufnahmeeinheiten 7, 8, 9 zueinander beabstandet sind, bewegt.

Der Zustand nach dem Bewegen des Unterteils 2 in Richtung des Oberteils 1 bzw. umgekehrt ist in Figur 8 in einer Figur 7 entsprechenden Ansicht dargestellt. Das Unterteil 2 ist hierbei in eine Ausnehmung des Oberteils 1 eingeführt, so dass der erste Teil 12 innerhalb des Haltegehäuses 3 liegt und von diesem umschlossen wird, während der zweite Teil 13 außerhalb des Haltegehäuses 3 liegt. In diesem zusammengeschobenen Zustand sind die Spitzen der Probenaufnahmeeinheiten 7, 8, 9 aus der Öffnung 10 ragend und bilden eine gemeinsame Saugspitze, die durch die Öffnung 10 zudem auch mechanisch fixiert ist. Das Unterteil 2 und das Oberteil 1 können somit in einer formschlüssigen bzw. kraftschlüssigen Verbindung miteinander verbunden sein. Zusätzlich kann ein Anschlag an dem Oberteil 1 oder dem Unterteil 2 vorgesehen sein, um die Translationsbewegung zwischen beiden Teilen 12 und 13 an einer definierten Position zu stoppen.

Es wird somit eine Probenaufnahmevorrichtung als Verbrauchsmaterial zum typischerweise einmaligen Verwenden zur Verfügung gestellt, die manuell oder automatisiert in einem einzigen Schritt passiv Teilproben bzw. Aliquots aus einer einzigen Probe aufnehmen kann. Die aufgenommenen Teilproben können danach weiteranalysiert werden. Dadurch, dass die verschiedenen Probenaufnahmeeinheiten 7, 8, 9 auch von dem Haltegehäuse 3 getrennt werden können, kann jede der Teilproben separiert weiterbehandelt und analysiert werden, wodurch ein Diagnosepotenzial verbessert wird. Durch die beschriebene Vorrichtung können auch geringe Probenmengen mit möglichst wenig Veränderung an der Probensubstanz aufgenommen werden, wobei eine große Homogenisierung der Teilproben durch Entnahme an einer einzigen Ursprungsprobe erreicht wird. Zudem wird, beispielsweise bei der Entnahme an einer Fingerspitze, eine unnötige Belastung des Patienten durch eine mehrfache Probenentnahme vermieden. Die Vorrichtung verzichtet durch eine entsprechende Ausgestaltung der Probenaufnahmeeinheiten 7, 8, 9, beispielsweise als Kapillaren, auf aktive Komponenten wie komplexe Mikrofluidik und kann ohne weitergehende kompliziertere Geräte gegebenenfalls auch einfach per Hand bedient werden.

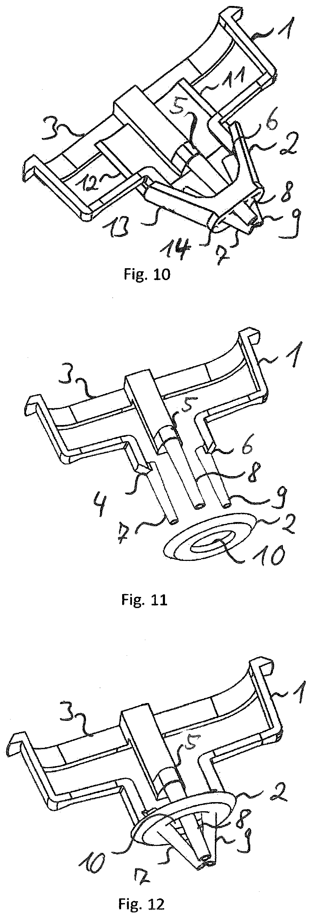

Figur 9 zeigt in einer Figur 5 entsprechenden Ansicht ein weiteres Ausführungsbeispiel des Unterteils 2, bei dem die Öffnung 10 nun durch ein mindestens die Öffnung 10 füllendes Säuberungselement 14 wie einen Schwamm, überdeckt ist, das innerhalb des Gehäuses 11 oder außerhalb des Gehäuses 11 angeordnet ist. Das Säuberungselement 14 kann alternativ oder zusätzlich auch im zweiten Teil 13 oder im ersten Teil 12 angeordnet sein. Das Säuberungselement 14 kann ebenfalls einteilig, also aus einem Stück bestehend bzw. monolithisch ausgebildet sein, es kann aber auch aus mehreren Einzelteilen ausgebildet sein, die allerdings auch miteinander verbundenen, beispielsweise zusammengeschweißt sein können. Das Säuberungselement 14 fährt beim Durchführen der beschriebenen Relativbewegung von Oberteil 1 und Unterteil 2 an einer Außenseite der Probenaufnahmeeinheiten 7, 8, 9 entlang und säubert diese. Dementsprechend wird auch nach Probenaufnahme beim Auseinanderziehen des Unterteils 2 und des Oberteils 1 eine Säuberung durchgeführt und die Probenaufnahmeeinheiten 7, 8, 9 liegen mit darin aufgenommener Probe und gesäuberten Außenseiten räumlich voneinander beabstandet vor. Figur 10 zeigt entsprechend den zusammengeschobenen Zustand in einer Figur 8 entsprechenden Ansicht.

Das volumenveränderliche Säuberungselement 14 ist besonders vorteilhaft einsetzbar, wenn ein Benutzer der Probenaufnahmevorrichtung kann spezielle Ausbildung für die Arbeit in einem Labor besitzt und dementsprechend nur unzureichend oder gar nicht mit Regeln zur sauberen Probenaufnahme vertraut ist. Bei Proben, die aufgrund weiterlaufender biologischer bzw. chemischer Prozesse zeitkritisch hinsichtlich der Analyse sind, beispielsweise Urin, kann eine schnellere Analyse durchgeführt werden, da eine Säuberung der Außenseite bereits beim Abstreifen durchgeführt wird. Durch das Säuberungselement 14 wird zudem auch eine Verschmutzung der Innenwand des Unterteils 2 und natürlich der Außenseiten der Probenaufnahmeeinheiten 7, 8, 9 vermieden, so dass die Reproduzierbarkeit aufgrund sauberer Probenbehälter erhöht wird und mit höherer Wahrscheinlichkeit nur die gewünschte Probe in den Probenaufnahmeeinheiten 7, 8, 9 enthalten ist und auch die Sicherheit für einen Benutzer erhöht wird, da ein unfreiwilliger Kontakt mit der Probe vermieden wird.

Figur 11 zeigt in einer Figur 7 entsprechenden perspektivischen Ansicht das bereits beschriebene Oberteil 1, auf das nun jedoch ein scheibenförmiges oder ringförmiges Unterteil 2 aufgesetzt werden soll. Das scheibenförmige Unterteil 2 weist hierzu eine Öffnung 10 auf, die wie zuvor mittig angeordnet ist, jedoch ist eine Höhe des Unterteils 2 nun deutlich geringer als seine Länge bzw. Breite, typischerweise beträgt die Höhe maximal 10 Prozent der Länge oder Breite. Die Probenaufnahmeeinheiten 7, 8, 9 werden entlang der Verbindungen 4, 5, 6 durch eine externe Kraft, beispielsweise manuell oder durch eine weitere Maschine, in die gewünschte Position bewegt, in der ihre Spitzen einander berühren, und durch Einbringen in die Öffnung 10, d. h. durch Aufsetzen des Unterteils 2 in dieser Position arretiert bzw. fixiert. Hierbei kann wiederum eine formschlüssige bzw. kraftschlüssige Verbindung zwischen dem Oberteil 1 und dem Unterteil 2 hergestellt werden. Figur 12 zeigt das Oberteil 1 mit dem aufgesetzten Unterteil 2 aus dem in Figur 11 beschriebenen Ausführungsbeispiel.

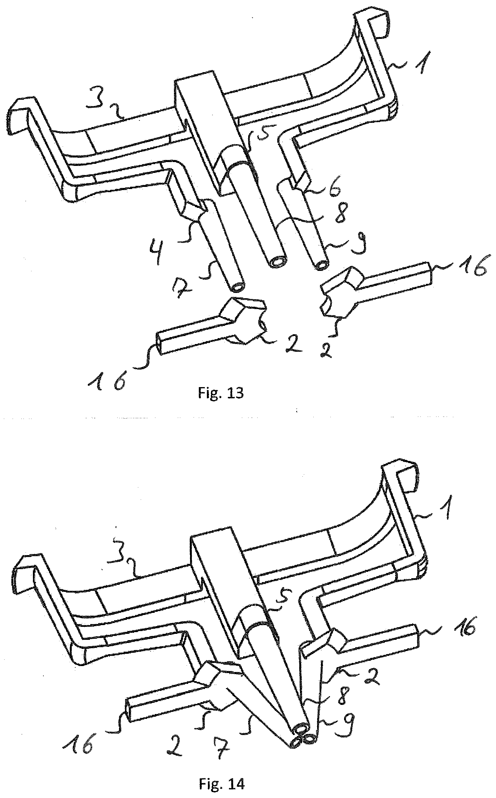

Als weitere Form einer Arretiervorrichtung kann auch eine Haltevorrichtung in Form von zwei Klemmen 16 als Unterteil 2 verwendet werden, wie in Figur 13 in einer Figur 7 entsprechenden perspektivischen Ansicht dargestellt. Die Probenaufnahmeeinheiten 7, 8, 9 werden hierbei wie bei dem in den Figuren 11 und 12 wiedergegebenen Ausführungsbeispiel durch eine externe Kraft in die gewünschte Position bewegt und durch die beiden Klemmen 16 in dieser Position fixiert, wie in Figur 14 in einer entsprechenden Ansicht dargestellt. Vorteilhaft ist an dieser Ausgestaltung insbesondere, dass verschiedene Abstände der Probenaufnahmeeinheiten 7, 8, 9 zueinander einstellbar sind. In weiteren Ausführungsbeispielen kann auch nur eine einzige Klemme 16 verwendet werden oder die beiden Klemmen können einteilig ausgestaltet sein, d. h. nur aus einem einzigen Teil bestehen.

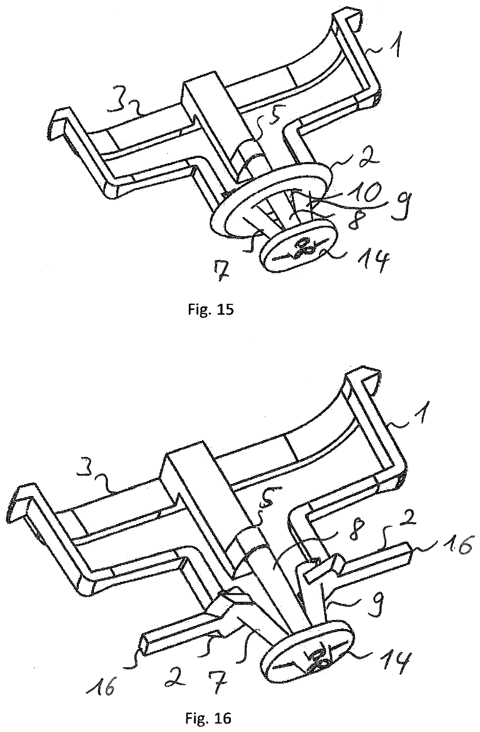

In den Figuren 15 und 16 sind die in den Figuren 12 und 14 dargestellten Ausführungsbeispiele wiedergegeben, wobei nun jedoch das Säuberungselement 14 auf die Spitzen der Probenaufnahmeeinheiten 7, 8, 9 aufgesetzt ist. Hierzu muss das Säuberungselement 14 nicht in das Unterteil 2 integriert sein, sondern kann auch als separates Bauteil vorliegen.

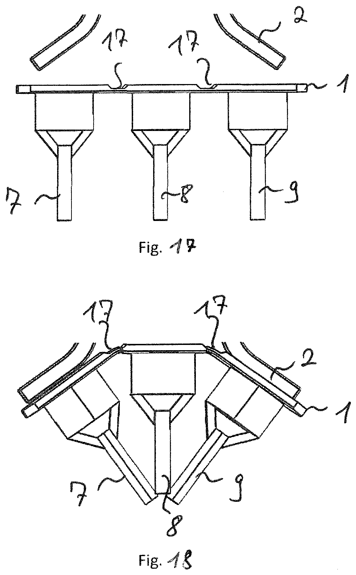

Figur 17 zeigt in einer schematischen Schnittansicht einen ersten Teil 1 einer Probenaufnahmevorrichtung (beispielsweise ein Unterteil) samt einem zweiten Teil 2 (beispielsweise einem Oberteil). Der erste Teil 1 weist die drei stabförmige Probenaufnahmeeinheiten 7, 8 und 9 auf, die nebeneinander angeordnet sind. Zwischen zwei benachbarten der Probeaufnahmeeinheiten 7, 8, und 9 ist jeweils mittig eine biegbare Verbindung 17 in Form eines Scharniers angeordnet. In dem in Figur 17 dargestellten Grundzustand sind die von den biegbaren Verbindungen 17 wegweisenden Enden oder Spitzen der Probenaufnahmeeinheiten 7, 8, und 9, d. h. die in Richtung einer aufzunehmenden Probe weisenden Enden, räumlich voneinander beabstandet.

In dem in Figur 17 gezeigten Ausführungsbeispiel sind die Probenaufnahmeeinheiten 7, 8 und 9 in einer Reihenanordnung angeordnet und die der Probe zugewandte Seite der Probenaufnahmeeinheiten 7, 8 und 9 sind in einer Ebene angeordnet. Die Probenaufnahmeeinheiten 7, 8 und 9 weisen eine identische Länge auf, können in weiteren Ausführungsbeispiele aber auch derart ausgebildet sein, dass zumindest eine der Probenaufnahmeeinheiten 7, 8 und 9 eine von den anderen Probenaufnahmeeinheiten 7, 8, 9 abweichende Länge aufweist. Ebenso ist die der Probe abgewandte Seite des ersten Teils 1 im Grundzustand in einer Ebene angeordnet. Die der Probe abgewandte Seite der Probenaufnahmeeinheiten 7, 8, 9 (d. h. die Seite, an der auch die biegbare Verbindung 17 angeordnet ist) ist geschlossen, während die Probenaufnahmeeinheiten 7, 8, 9 als Kapillaren ausgebildet sind und an ihrer der Probe zugewandten Seite eine Öffnung aufweisen. Die Anzahl der Probenaufnahmeeinheiten 7, 8, 9 ist zudem variabel und kann neben den dargestellten drei Probenaufnahmeeinheiten 7, 8, 9 auch nur zwei Probenaufnahmeeinheiten oder mehr als zwei Probenaufnahmeeinheiten, beispielsweise bis zu zehn Probenaufnahmeeinheiten, umfassen. Die Länge der Probenaufnahmeeinheiten 7, 8, 9 liegt in der Regel zwischen 1 mm und 20 mm, vorzugsweise im Bereich von 8 mm und 14 mm.

Die dargestellten Probenaufnahmeeinheiten 7, 8, 9 sind jeweils als Kapillaren für die Aufnahme von flüssigen Proben, beispielsweise Tropfen einer bestimmten zu analysierenden Flüssigkeit wie Blutstropfen von einer Fingerspitze, im Bereich von Millilitern ausgebildet und können vorzugsweise Probenvolumen von 0,25 µl bis 25 µl, besonders vorzugsweise 1 µl bis 20 µl aufnehmen. Die Probenaufnahmeeinheiten 7, 8, 9 weisen, unabhängig von der genauen Anzahl der Probenaufnahmeeinheiten 7, 8, 9, in der Regel ein jeweils identisches Innenvolumen bzw. jeweils einen innenliegenden Hohlraum mit jeweils identischem Volumen auf, d. h. es kann in jeder der Probenaufnahmeeinheiten 7, 8, 9 ein gleich großes Probenvolumen aufgenommen werden. In weiteren Ausführungsbeispielen kann aber auch das Probenvolumen wenigstens einer der Probenaufnahmeeinheiten 7, 8, 9 sich von dem Volumen der anderen Probenaufnahmeeinheiten 7, 8, 9 unterscheiden. Es können aber auch als Festkörper, beispielsweise als Granulat, Pulver oder sonstiges Schüttgut, vorliegende Proben oder gelförmige Proben aufgenommen werden. Die Probeaufnahmeeinheiten 7, 8, 9 sind in dem gezeigten Ausführungsbeispiel zwar starr ausgeführt, können in weiteren Ausführungsbeispielen aber auch selbst biegbar sein.

Der zweite Teil 2 ist in dem in Figur 17 abgebildeten Ausführungsbeispiel aus dem gleichen Werkstoff wie der erste Teil 1, kann in weiteren Ausführungsbeispielen aber auch aus einem anderen Werkstoff gebildet sein. Der zweite Teil 2 weist eine Arretiervorrichtung auf, die derart ausgebildet ist, dass die biegbare Verbindung 17 beim Aufsetzen des ersten Teils 1 mit seiner den Probenaufnahmeeinheiten 7, 8, 9 abgewandten Seite auf den zweiten Teil 2 durch die Form der Arretiervorrichtung, im dargestellten Beispiel eine trichterförmige Aussparung, gebogen wird und die Probenaufnahmeeinheiten 7, 8, 9 zueinander abgewinkelt werden.

Wie in Figur 18 in einer Figur 17 entsprechenden Ansicht dargestellt, sind im zusammengefügten Zustand der erste Teil 1 und der zweite Teil 2 in direktem Kontakt miteinander und die in Richtung der aufzunehmenden Probe (oder zumindest Teilvolumina davon bzw. ein Aliquot) weisenden Spitzen bzw. Enden der Probenaufnahmeeinheiten 7, 8, 9 in direktem Kontakt miteinander. Im Schnittpunkt der Längsachsen der stabförmigen Probenaufnahmeeinheiten 7, 8, 9 kann die aufzunehmende Probe, typischerweise eine Flüssigkeit, angeordnet sein und von dort in die Kapillaren gelangen. Durch das formschlüssige Verbinden von erstem Teil 1 und zweitem Teil 2 sind die Probenaufnahmeeinheiten 7, 8, 9 in ihrer Position fixiert.

Die Probenaufnahmeeinheiten 7, 8, 9 sind wie schon erläutert länglich bzw. stabförmig ausgebildet und in ihrem Inneren hohl, d. h. Kapillaren oder Hohlzylinder bzw. Röhren. In weiteren Ausführungsbeispielen können die Probenaufnahmeeinheiten 7, 8, 9 oder zumindest eine der Probenaufnahmeeinheiten 7, 8, 9 aber auch als Tupfer, Bürste, Spatel, Spachtelmesser, Nadel, Membran, Stab, schraubenförmiger Stab, Faser, Hohlfaser, Schaufel, Spatel, Löffel, Schwamm oder Schaum ausgebildet sein. Zudem müssen nicht alle der Probenaufnahmeeinheiten 7, 8, 9 vom gleichen Typ sein, es kann auch vorgesehen sein, dass wenigstens eine der Probenaufnahmeeinheiten 7, 8, 9 einen von den anderen Probenaufnahmeeinheiten 7, 8, 9 abweichenden Typ aufweist. An ihrer der biegbaren Verbindung 17 abgewandten und einer aufzunehmenden und nachfolgend zu analysierenden Probe zugewandten Seite weisen die Probenaufnahmeeinheiten 7, 8, 9 in dem dargestellten Ausführungsbeispiel jeweils einen geringeren Durchmesser als an ihrer der biegbaren Verbindung 17 zugewandten Seite auf.

Über die flexiblen, d. h. biegbaren und dehnbaren Verbindungen 17 können die Probeaufnahmeeinheiten 7, 8, 9 in geringem Maße geknickt bzw. rotiert werden, die Verbindungen 17 sind also scharnierförmig ausgestaltet. Zudem ist es auch möglich, durch ein Biegen über den jeweiligen Biegeradius hinaus die Probenaufnahmeeinheiten 7, 8, 9 voneinander zu trennen, d. h. die Verbindungen 17 aufzutrennen. Es kann auch vorgesehen sein, dass die Verbindungen 17 oder zumindest eine oder zwei der Verbindungen 17 derart ausgestaltet sind, dass sie bei einem Entfernen des ersten Teils 1 von dem zweiten Teil 2 wieder in die ursprüngliche Position, d. h. die in Figur 1 wiedergegebene Stellung zurückbewegt werden.

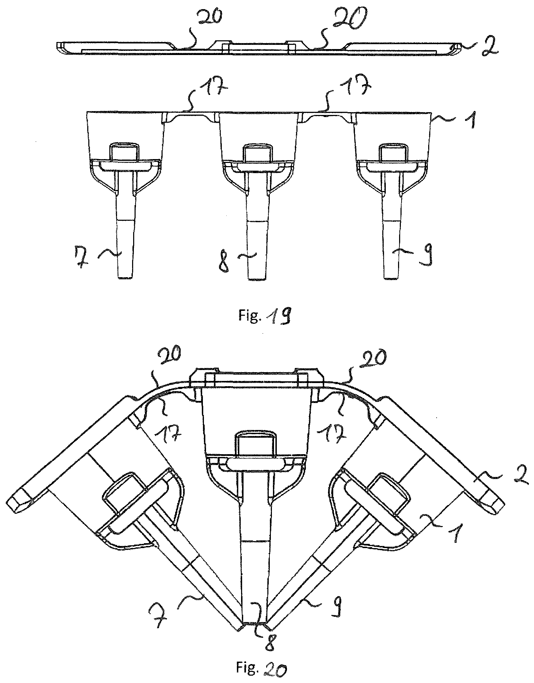

In Figur 19 ist in einer Figur 17 entsprechenden seitlichen Ansicht ein weiteres Ausführungsbeispiel gezeigt, bei dem der erste Teil 1 drei stabförmige Probenaufnahmeeinheiten 7, 8 und 9 aufweist, die nebeneinander angeordnet sind. Zwischen zwei benachbarten der Probeaufnahmeeinheiten 7, 8 und 9 ist wiederum jeweils mittig eine biegbare Verbindung 17 in Form eines Scharniers angeordnet. Der zweite Teil 2 ist in diesem Fall als biegbare Platte ausgestaltet, an der korrespondierend zu den biegbaren Verbindungen 17 biegbare Bereiche 20 angeordnet sind, die die gleichen mechanischen Eigenschaften wie die biegbaren Verbindungen 17 des ersten Teils 1 aufweisen können. In Figur 20 ist in einer Figur 18 entsprechenden Ansicht die Probenaufnahmevorrichtung im zusammengesetzten Zustand gezeigt, d. h. der erste Teil 1 und der zweite Teil 2 sind miteinander verbunden, wobei die biegbaren Verbindungen 17 und die biegbaren Bereiche 20 jeweils extern gebogen sind und durch Reibung, eine Klebeverbindung oder eine Kombination von beidem der erste Teil und der zweite Teil zusammengehalten werden.

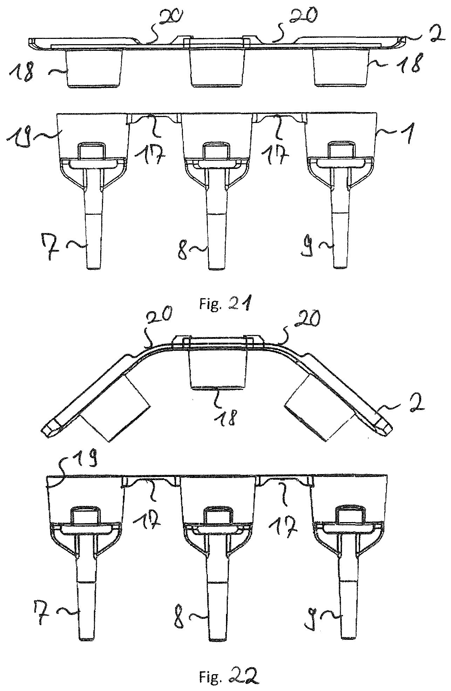

Figur 21 zeigt in einer den Figuren 1 und 3 entsprechenden Ansicht ein weiteres Ausführungsbeispiel, bei dem der zweite Teil im Gegensatz zu dem in Figur 3 dargestellten Ausführungsbeispiel bolzenförmige Vorsprünge 18 aufweist, die in korrespondierende Ausnehmungen des ersten Teils 1 eingebracht werden können. Figur 22 zeigt den vorgeformten zweiten Teil 2, auf den in der Folge der erste Teil 1 aufsetzbar und wiederum mit einer Klebeverbindung und bzw. oder eine durch Reibung gehaltene Verbindung zwischen den Vorsprüngen 18 und den Ausnehmungen, in die sie eingebracht werden, befestigt werden.

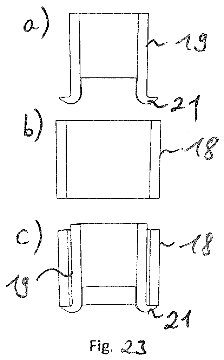

Figur 23 zeigt in einer seitlichen Schnittansicht eine mögliche Ausgestaltung dieser Ausnehmung 19 in Figur 23b), während in Figur 23a) der bolzenförmige Vorsprung 18 als Hohlkörper gezeigt ist. In Figur 23c) sind beide Elemente zusammengesetzt, wobei nun ein Anschlag 21 an der Ausnehmung 19 die Bewegung der Aussparung 18 begrenzt. Es wird in dem dargestellten Ausführungsbeispiel also der zweite Teil 2 in den ersten Teil 1 eingeführt.

Es wird somit eine Probenaufnahmevorrichtung als Verbrauchsmaterial zum typischerweise einmaligen Verwenden zur Verfügung gestellt, die manuell oder automatisiert in einem einzigen Schritt passiv Teilproben bzw. Aliquots aus einer einzigen Probe aufnehmen kann. Die aufgenommenen Teilproben können danach weiteranalysiert werden. Dadurch, dass die verschiedenen Probenaufnahmeeinheiten 7, 8, 9 auch voneinander getrennt werden können, kann jede der Teilproben separiert weiterbehandelt und analysiert werden, wodurch ein Diagnosepotenzial verbessert wird. Durch die beschriebene Vorrichtung können auch geringe Probenmengen mit möglichst wenig Veränderung an der Probensubstanz aufgenommen werden, wobei eine große Homogenisierung der Teilproben durch Entnahme an einer einzigen Ursprungsprobe erreicht wird. Zudem wird, beispielsweise bei der Entnahme an einer Fingerspitze, eine unnötige Belastung des Patienten durch eine mehrfache Probenentnahme vermieden. Die Vorrichtung verzichtet durch eine entsprechende Ausgestaltung der Probenaufnahmeeinheiten 7, 8, 9, beispielsweise als Kapillaren, auf aktive Komponenten wie komplexe Mikrofluidik und kann ohne weitergehende kompliziertere Geräte gegebenenfalls auch einfach per Hand bedient werden.

Die beschriebene Vorrichtung bzw. ein entsprechendes Probenaufnahmeverfahren mit der genannten Probenaufnahmevorrichtung können im medizinischen oder tiermedizinischen Bereich, aber auch industriell bzw. zur Analyse von Lebensmitteln wie Getränken oder ökologische Analysen eingesetzt werden. Die beschriebene Vorrichtung ist insbesondere vorteilhaft, da sowohl Probenverlust (der generell vermieden werden sollte), ein Verschmieren der Probe (was zu fehlerhaften Messergebnissen führen kann) und eine Exposition des Benutzers mit potentiell infektiösen oder anderweitig schädlichen Proben komplett oder weitestgehend vermieden wird.

## Patentansprüche

1. Probenaufnahmevorrichtung mit
einem ersten Teil (1) und einem zweiten Teil (2), wobei
der erste Teil (1) mindestens zwei über eine biegbare Verbindung (17) miteinander verbundene stabförmige Probenaufnahmeeinheiten (7, 8, 9) aufweist,
deren von der biegbaren Verbindung (17) wegweisende Enden in einem Grundzustand räumlich voneinander beabstandet sind,
wobei der zweite Teil (2) eine Arretiervorrichtung aufweist, die derart ausgebildet ist, dass die biegbare Verbindung (17) beim Aufsetzen des ersten Teils (1) mit seiner den Probenaufnahmeeinheiten (7, 8, 9) abgewandten Seite auf den zweiten Teil (2) durch die Form der Arretiervorrichtung gebogen wird, **dadurch gekennzeichnet, dass** die Enden der mindestens zwei Probenaufnahmevorrichtungen (7, 8, 9) zueinander geführt werden können, so dass
die in Richtung einer Probe weisend angeordneten Seiten der mindestens zwei Probenaufnahmeeinheiten (7, 8, 9) unmittelbar nebeneinander angeordnet sind, durch die Arretiervorrichtung in dieser Position arretiert sind und eine einzige Probe in die mindestens zwei Probenaufnahmeeinheiten (7, 8, 9) oder mit den mindestens zwei Probenaufnahmeeinheiten (7, 8, 9) aufnehmbar ist.

2. Probenaufnahmevorrichtung nach Anspruch 1, **dadurch gekennzeichnet, dass** die biegbare Verbindung (17) eine scharnierförmige Verbindung ist.

3. Probenaufnahmevorrichtung nach Anspruch 1 oder Anspruch 2, **dadurch gekennzeichnet, dass** die biegbare Verbindung (17) mittig zwischen den zwei Probenaufnahmeeinheiten (7, 8, 9) angeordnet ist.

4. Probenaufnahmevorrichtung nach einem der vorhergehenden Ansprüche, **dadurch gekennzeichnet, dass** zumindest eine der Probenaufnahmeeinheiten (7, 8, 9) als Kapillare, Röhre, Faser, vorzugsweise Hohlfaser, Tupfer, Bürste, Spatel, Spachtelmesser, Nadel, Membran, Schaufel, Löffel, Schwamm, vorzugsweise fester Schwamm, oder Stab, vorzugsweise schraubenförmiger Stab, oder mit einer Kapillare, einer Röhre, einer Faser, vorzugsweise einer Hohlfaser, einem Tupfer, einer Bürste, einem Spatel, einem Spachtelmesser, einer Nadel, einer Membran, einer Schaufel, einem Löffel, einem Schwamm, vorzugsweise einem festen Schwamm, oder einem Stab, vorzugsweise einem schraubenförmigen Stab, ausgebildet ist.

5. Probenaufnahmevorrichtung nach einem der vorhergehenden Ansprüche **dadurch gekennzeichnet, dass** der erste Teil (1) genau drei Probenaufnahmeeinheiten (7, 8, 9) aufweist, die in einer Reihenanordnung angeordnet sind und von denen die der Probe zugewandten Seiten der Probenaufnahmeeinheiten (7, 8, 9) im Grundzustand in einer Ebene angeordnet sind, wobei die mittig zwischen zwei Probenaufnahmeeinheiten (7, 9) angeordnete der Probenaufnahmeeinheiten (8) über jeweils eine biegbare Verbindung (17) mit der jeweils benachbarten Probenaufnahmeeinheit (7, 9) verbunden ist.

6. Probenaufnahmevorrichtung nach einem der vorhergehenden Ansprüche, **dadurch gekennzeichnet, dass** der erste Teil (1) an einer der in Richtung der Probe weisend angeordneten Spitzen der mindestens zwei Probenaufnahmeeinheiten (7, 8, 9) abgewandten Seite geschlossen ausgebildet ist.

7. Probenaufnahmevorrichtung nach einem der vorhergehenden Ansprüche, **dadurch gekennzeichnet, dass** die Arretiervorrichtung des zweiten Teils (2) eine trichterförmige Form zum Aufnehmen des ersten Teils (1) aufweist.

8. Probenaufnahmevorrichtung nach einem der vorhergehenden Ansprüche, **dadurch gekennzeichnet, dass** der erste Teil (1) und der zweite Teil (2) formschlüssig oder kraftschlüssig miteinander verbindbar sind.

9. Probenaufnahmevorrichtung nach einem der vorhergehenden Ansprüche, **dadurch gekennzeichnet, dass** der erste Teil (1) mit den mindestens zwei Probenaufnahmeeinheiten (7, 8, 9) aus einem für elektromagnetische Strahlung im optisch sichtbaren Wellenlängenbereich durchlässigen Kunststoff ausgebildet ist.

10. Probenaufnahmevorrichtung nach einem der vorhergehenden Ansprüche, **dadurch gekennzeichnet, dass** das Haltegehäuse (3) an einer der in Richtung der Probe weisend angeordneten Seiten der mindestens zwei Probenaufnahmeeinheiten (7, 8, 9) abgewandten Seite geschlossen ausgebildet ist.

11. Verfahren zur Probenaufnahme mittels der Probenaufnahmevorrichtung nach einem der Ansprüche 1-10, bei dem die biegbare Verbindung (17) beim Aufsetzen des ersten Teils (1) mit seiner den Enden der Probenaufnahmeeinheiten (7, 8, 9) abgewandten Seite auf den zweiten Teil (2) durch die Form der Arretiervorrichtung gebogen werden und die Enden der mindestens zwei Probenaufnahmeeinheiten (7, 8, 9) zueinander geführt werden, so dass die in Richtung einer Probe weisend angeordneten Enden der mindestens zwei Probenaufnahmeeinheiten (7, 8, 9) unmittelbar nebeneinander angeordnet sind, durch die Arretiervorrichtung in dieser Position arretiert sind und eine einzige Probe in die mindestens zwei Probenaufnahmeeinheiten (7, 8, 9) oder mit den mindestens zwei Probenaufnahmeeinheiten (7, 8, 9) aufnehmbar ist.

## Claims

1. A sample receiving device comprising
a first part (1) and a second part (2), wherein
the first part (1) has at least two rod-shaped sample receiving units (7, 8, 9) connected to one another via a bendable connection (17),
whose ends facing away from the bendable connection (17) are spatially spaced apart from one another in a basic state,
wherein the second part (2) has a locking device which is designed in such a way that, when the first part (1) is placed with its side facing away from the sample receiving units (7, 8, 9) onto the second part (2), the bendable connection (17) is bent by the shape of the locking device, **characterized in that** the ends of the at least two sample receiving devices (7, 8, 9) can be guided towards one another so that
the sides of the at least two sample receiving units (7, 8, 9) facing in the direction of a sample are arranged directly next to one another, are locked in this position by the locking device and a single sample can be received in the at least two sample receiving units (7, 8, 9) or with the at least two sample receiving units (7, 8, 9).

2. The sample receiving device according to claim 1, **characterized in that** the bendable connection (17) is a hinge-shaped connection.

3. Sample receiving device according to claim 1 or claim 2, **characterized in that** the bendable connection (17) is arranged centrally between the two sample receiving units (7, 8, 9).

4. The sample receiving device according to any one of the preceding claims, **characterized in that** at least one of the sample receiving units (7, 8, 9) is in the form of a capillary, a tube, a fiber, preferably a hollow fiber, a swab, a brush, a spatula, a spatula knife, a needle, a membrane, a scoop, a spoon, a sponge, preferably a solid sponge, or a rod, preferably a helical rod, or has a capillary, a tube, a fiber, preferably a hollow fiber, a swab, a brush, a spatula, a spatula knife, a needle, a membrane, a scoop, a spoon, a sponge, preferably a solid sponge, or a rod, preferably a helical rod.

5. The sample receiving device according to one of the preceding claims, **characterized in that** the first part (1) has precisely three sample receiving units (7, 8, 9) which are arranged in a row arrangement and of which the sides of the sample receiving units (7, 8, 9) facing the sample are arranged in a plane in the basic state, the one of the sample receiving units (8) arranged centrally between two sample receiving units (7, 9) being connected to the respectively adjacent sample receiving unit (7, 9) via in each case one bendable connection (17).

6. The sample receiving device according to one of the preceding claims, **characterized in that** the first part (1) is formed closed at a side facing away from the tips of the at least two sample receiving units (7, 8, 9) arranged facing in the direction of the sample.

7. The sample receiving device according to any one of the preceding claims, **characterized in that** the locking device of the second part (2) is funnel-shaped for receiving the first part (1).

8. The sample receiving device according to one of the preceding claims, **characterized in that** the first part (1) and the second part (2) can be connected to one another in a form-fitting or force-fitting manner.

9. The sample receiving device according to one of the preceding claims, **characterized in that** the first part (1) with the at least two sample receiving units (7, 8, 9) is formed from a plastic material which is transparent to electromagnetic radiation in the optically visible wavelength range.

10. The sample receiving device according to one of the preceding claims, **characterized in that** the holding housing (3) is formed closed at a side facing away from the sides of the at least two sample receiving units (7, 8, 9) arranged facing in the direction of the sample.

11. A method for receiving samples by means of the sample receiving device according to one of the claims 1-10, in which the bendable connection (17), when the first part (1) is placed with its side facing away from the ends of the sample receiving units (7, 8, 9) onto the second part (2), is bent by the shape of the locking device and the ends of the at least two sample receiving units (7, 8, 9) are guided towards each other, so that the ends of the at least two sample receiving units (7, 8, 9) arranged pointing in the direction of a sample are arranged directly next to each other, are locked in this position by the locking device and a single sample can be received in the at least two sample receiving units (7, 8, 9) or with the at least two sample receiving units (7, 8, 9).

## Revendications

1. Dispositif de réception d'échantillon avec
une première partie (1) et une deuxième partie (2), dans lequel
la première partie (1) présente au moins deux unités de réception d'échantillon (7, 8, 9) en forme de tige reliées entre elles par une liaison flexible (17),
dont les extrémités s'écartant de la liaison flexible (17) dans un état de base sont séparées spatialement l'une de l'autre,
dans lequel la deuxième partie (2) présente un dispositif d'arrêt qui est réalisé de telle sorte que, lors de la mise en place de la première partie (1) avec son côté opposé aux unités de réception d'échantillon (7, 8, 9) sur la deuxième partie (2), la liaison flexible (17) est fléchie par la forme du dispositif d'arrêt, **caractérisé en ce que** les extrémités des au moins deux dispositifs de réception d'échantillon (7, 8, 9) peuvent être guidées l'une vers l'autre, de sorte que
les côtés des au moins deux unités de réception d'échantillon (7, 8, 9) disposés orientés en direction d'un échantillon sont disposés directement côte à côte, sont arrêtés dans cette position par le dispositif d'arrêt et un seul échantillon peut être reçu dans les au moins deux unités de réception d'échantillon (7, 8, 9) ou avec les au moins deux unités de réception d'échantillon (7, 8, 9).

2. Dispositif de réception d'échantillon selon la revendication 1, **caractérisé en ce que** la liaison flexible (17) est une liaison en forme de charnière.

3. Dispositif de réception d'échantillon selon la revendication 1 ou la revendication 2, **caractérisé en ce que** la liaison flexible (17) est disposée au centre entre les deux unités de réception d'échantillon (7, 8, 9).

4. Dispositif de réception d'échantillon selon l'une quelconque des revendications précédentes, **caractérisé en ce qu'**au moins l'une des unités de réception d'échantillon (7, 8, 9) est réalisée sous la forme d'un capillaire, d'un tube, d'une fibre, de préférence d'une fibre creuse, d'un écouvillon, d'une brosse, d'une spatule, d'un couteau à enduire, d'une aiguille, d'une membrane, d'une pelle, d'une cuillère, d'une éponge, de préférence une éponge solide, ou d'une tige, de préférence une tige hélicoïdale, ou avec un capillaire, un tube, une fibre, de préférence une fibre creuse, un écouvillon, une brosse, une spatule, un couteau à enduire, une aiguille, une membrane, une pelle, une cuillère, une éponge, de préférence une éponge solide, ou une tige, de préférence une tige hélicoïdale.

5. Dispositif de réception d'échantillon selon l'une quelconque des revendications précédentes, **caractérisé en ce que** la première partie (1) présente exactement trois unités de réception d'échantillon (7, 8, 9) qui sont disposées en une rangée et dont les côtés des unités de réception d'échantillon (7, 8, 9) tournés vers l'échantillon à l'état de base sont disposés dans un plan, dans lequel celle des unités de réception d'échantillon (8) disposée au centre entre deux unités de réception d'échantillon (7, 9) est reliée à l'unité de réception d'échantillon (7, 9) respectivement voisine par l'intermédiaire d'une liaison flexible (17).

6. Dispositif de réception d'échantillon selon l'une quelconque des revendications précédentes, **caractérisé en ce que** la première partie (1) est réalisée de manière fermée sur un côté opposé aux pointes disposées orientées en direction de l'échantillon des au moins deux unités de réception d'échantillon (7, 8, 9).

7. Dispositif de réception d'échantillon selon l'une quelconque des revendications précédentes, **caractérisé en ce que** le dispositif d'arrêt de la deuxième partie (2) présente une forme d'entonnoir pour recevoir la première partie (1).

8. Dispositif de réception d'échantillon selon l'une quelconque des revendications précédentes, **caractérisé en ce que** la première partie (1) et la deuxième partie (2) peuvent être reliées entre elles par complémentarité de forme ou à force.

9. Dispositif de réception d'échantillon selon l'une quelconque des revendications précédentes, **caractérisé en ce que** la première partie (1) avec les au moins deux unités de réception d'échantillon (7, 8, 9) est réalisée à partir d'un plastique transparent au rayonnement électromagnétique dans la plage des longueurs d'onde optiquement visibles.

10. Dispositif de réception d'échantillon selon l'une quelconque des revendications précédentes, **caractérisé en ce que** le boîtier de maintien (3) est réalisé de manière fermée sur un côté opposé aux côtés des au moins deux unités de réception d'échantillon (7, 8, 9) disposés orientés en direction de l'échantillon.

11. Procédé de réception d'échantillon au moyen du dispositif de réception d'échantillon selon l'une quelconque des revendications 1 à 10, selon lequel la liaison flexible (17), lors de la mise en place de la première partie (1) avec son côté opposé aux extrémités des unités de réception d'échantillon (7, 8, 9) sur la deuxième partie (2), est fléchie par la forme du dispositif d'arrêt et les extrémités des au moins deux unités de réception d'échantillon (7, 8, 9) sont guidées l'une vers l'autre, de sorte que les extrémités des au moins deux unités de réception d'échantillon (7, 8, 9) disposées orientées en direction d'un échantillon sont disposées directement côte à côte, sont arrêtées dans cette position par le dispositif d'arrêt et un seul échantillon peut être reçu dans les au moins deux unités de réception d'échantillon (7, 8, 9) ou avec les au moins deux unités de réception d'échantillon (7, 8, 9).
